# EUROPÄISCHE PATENTANMELDUNG

(11) **EP 3 248 960 A1**
(43) Veröffentlichungstag der Anmeldung: **29.11.2017**
(21) Anmeldenummer: 16171310.2
(22) Anmeldetag: 25.05.2016
(51) Int. Cl.: C07C 209/52

(54) **DESTILLATIVE AUFREINIGUNG EINES PRODUKTGEMISCHES ANFALLEND BEI DER ISOPHORONDIAMINSYNTHESE MIT HILFE EINER TRENNWANDKOLONNE**

(71) Anmelder: Evonik Degussa GmbH, 45128 Essen (DE)
(72) Erfinder: RITTSTEIGER, Anne, 59399 Olfen (DE); RÜFER, Alexander Martin, 45657 Recklinghausen (DE); STREUKENS, Guido, 42111 Wuppertal (DE); KOHLSTRUK, Stephan, 45966 Gladbeck (DE); ORSCHEL, Matthias, 48161 Münster (DE); MENDORF, Matthias, 44141 Dortmund (DE); HILLER, Christoph, 48249 Dülmen (DE); HENGSTERMANN, Axel, 48308 Senden (DE); MÜLLER, Anja, 44135 Dortmund (DE)

(57) **Zusammenfassung**

Die Erfindung betrifft die Feinreinigung von Isophorondiamin (IPDA) mit Hilfe einer Trennwandkolonne.

## Beschreibung

Die Erfindung betrifft die Feinreinigung von Isophorondiamin (IPDA) mit Hilfe einer Trennwandkolonne.

Die Herstellung von IPDA durch aminierende Hydrierung von Isophoronnitril (IPN) ist bekannt und wurde bereits mehrfach beschrieben.

Im einfachsten Fall (US 3,352,913) wird IPN in Gegenwart von Wasserstoff und eines Überschusses an Ammoniak an einem Cobaltkatalysator zur Reaktion gebracht. Zunächst bildet sich aus IPN und Ammoniak durch Wasserabspaltung das Isophoronnitrilimin, IPNI, das nachfolgend zum IPDA hydriert wird.

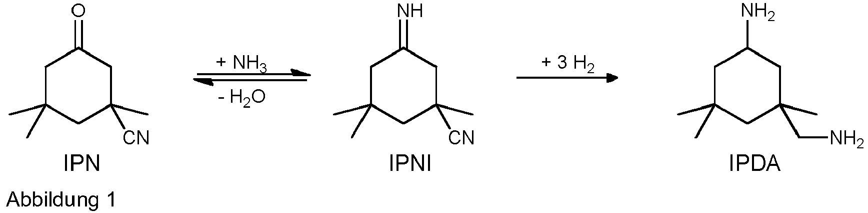

Weiterhin sind Verfahren zur Herstellung von Isophorondiamin aus CN 104230721A, EP 2649042A und WO 2012126869A bekannt.

Isophorondiamin wird gemäß der EP 2649042A in einer ein- oder zweistufigen Reaktion aus Isophoronnitril hergestellt. Dabei wird Isophoronnitril zunächst mit Ammoniak zu Isophoronnitrilimin iminiert. Im zweiten Schritt wird dieses zu Isophorondiamin hydriert. Die an die Reaktion angeschlossene Aufreinigung gliedert sich ebenfalls in zwei Schritte. Zunächst werden in mehreren Destillationskolonnen die Leichtsieder abgetrennt, dazu gehören Wasserstoff, Inertgase, Ammoniak und leichtsiedende Verunreinigungen (Leichtsiederabtrennung). In einem letzten Schritt wird dann das Reinisophorondiamin durch zwei Vakuumdestillationskolonnen gewonnen. Die erste Kolonne dient wiederum der Abtrennung von noch enthaltenen leichter siedenden Nebenprodukten. In der zweiten Kolonne wird das Isophorondiamin rein über Kopf gewonnen und so von den organischen Rückständen getrennt.

Aufgabe der vorliegenden Erfindung ist es, ein einfaches Verfahren zur Feinreinigung von Isophorondiamin zu finden mit reduziertem Energiebedarf.

Überraschenderweise wurde gefunden, dass sich die Feinreinigung in mindestens einer sogenannten Trennwandkolonne, mit einem geringen Energiebedarf durchführen lässt.

Gegenstand der Erfindung ist ein Verfahren zur Feinreinigung von Isophorondiamin aus der Herstellung von Isophorondiamin durch aminierende Hydrierung von Isophoronnitril in Anwesenheit von mindestens Ammoniak, Wasserstoff, eines Hydrierkatalysator und gegebenenfalls weiteren Zusätzen und in An- oder Abwesenheit von organischen Lösungsmitteln, wobei ein Roh-Isophorondiamin erhalten wird,
dadurch gekennzeichnet, dass das Roh-Isophorondiamin in mindestens einer Trennwandkolonne einer Feinreinigung unterworfen wird.

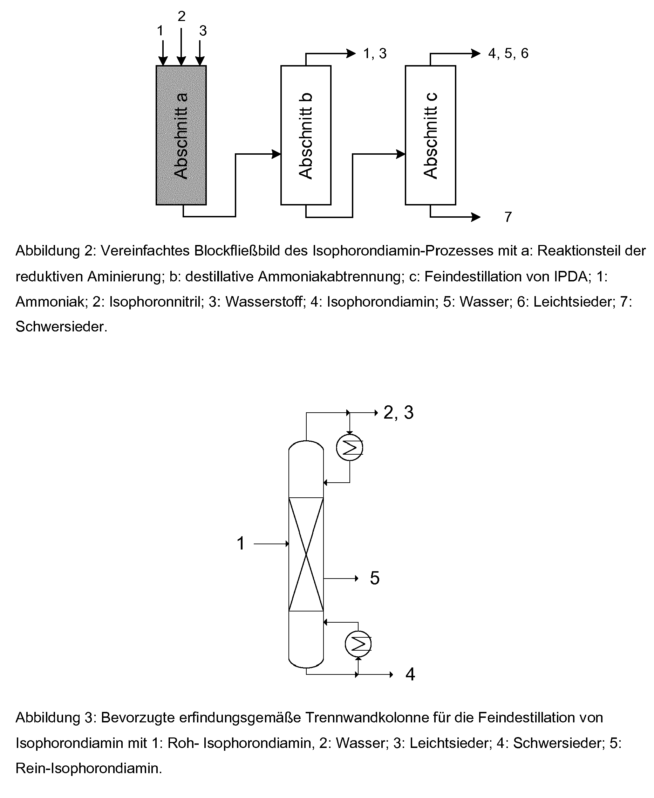

Der gesamte Prozess zur Herstellung von reinem IPDA gliedert sich in drei Abschnitte (siehe Abbildung 2). In Abschnitt a erfolgt die Reaktion durch aminierende Hydrierung von Isophoronnitril in einem ein- oder mehrstufigen Prozess unter Anwesenheit von mindestens Ammoniak, Wasserstoff und eines Katalysators. In Abschnitt b erfolgt die destillative Abtrennung von Ammoniak und Wasserstoff zur Gewinnung von Roh-IPDA. Die Destillation kann in einer oder mehreren Kolonnen durchgeführt werden. In Abschnitt c erfolgt gemäß dem Stand der Technik die Feinreinigung vom Roh-IPDA durch destillative Abtrennung von IPDA, Wasser, Leichtsieder und Schwersieder.
Erfindungsgemäß erfolgt in Abschnitt c die Feinreinigung von Roh-IPDA durch destillative Abtrennung von IPDA, Wasser, Leichtsieder und Schwersieder in einer oder mehreren Trennwandkolonnen, bevorzugt in einer Trennwandkolonne, siehe Abbildung 3.

Das Roh-IPDA weißt im Allgemeinen die folgende Zusammensetzung in Gewichts-% (Gew.-%) auf:

| | |
|---|---|
| IPDA | 75-100 Gew.-% |
| Wasser | 0-15 Gew.-% |
| Leichtsieder | 0-6 Gew.-% |
| Schwersieder | 0-6 Gew.-% |

Leichtsieder sind dabei so definiert, dass es sich hierbei um Nebenprodukte aus dem Herstellungsprozess handelt mit einem niedrigeren Siedepunkt als IPDA. Schwersieder sind dabei so definiert, dass es sich hierbei um Nebenprodukte aus dem Herstellungsprozess handelt mit einem höheren Siedepunkt als IPDA.

In einer Trennwandkolonne kann die Trennleistung von zwei oder mehreren in Reihe geschalteten Kolonnen vereint werden.

Bevorzugt wird wie folgt verfahren, siehe Abbildung 3, wobei
- über den Kopf der Trennwandkolonne die Abtrennung von Wasser und Leichtsiedern erfolgt,
- aus dem Sumpf der Trennwandkolonne die Schwersieder abgetrennt werden,
- das Rein- Isophorondiamin aus mindestens einem Seitenstrom der Trennwandkolonne gewonnen wird.

Die Trennwandkolonne weist folgende Rahmenbedingungen auf:

| | |
|---|---|
| Druck | 10-200 mbar |
| Sumpftemperatur | 120-250°C |
| Kopftemperatur | 40-120°C |
| Seitenstromtemperatur | 100-200°C |
| Theoretische Stufen | 10-130 |

Die Reinheit des so erfindungsgemäß erhaltenen Rein-Isophorondiamins soll mindestens 98 Gew.-% betragen.

### Beispiele

### Beispiel 1: Vergleichsbeispiel

Die Simulation der Destillation erfolgte über Aspen Plus. Im Vergleichsbeispiel wurde ein Aufbau, bestehend aus zwei Destillationskolonnen gewählt. Am Kopf der ersten Kolonne wurde ein Dekanter zur Trennung der beiden flüssigen Phasen verwendet, wobei nur die organische Phase als Rücklauf für die Kolonne verwendet wurde. Die erste Kolonne wies dabei 36 und die zweite Kolonne 15 theoretische Trennstufen auf, bei Rücklauf-zu-Feed-Verhältnissen von 1,1 (erste Kolonne) und 1,4 (zweite Kolonne). Hierbei wurde der Kolonnendruck der ersten Kolonne auf 110 mbar und der der zweiten Kolonne auf 80 mbar festgesetzt.
Der eingesetzte Feedstrom hatte folgende Zusammensetzung in Gewichts-% (Gew.-%):

| | |
|---|---|
| IPDA | 88,6 Gew.-% |
| Wasser | 9,1 Gew.-% |
| Leichtsieder | 1,2 Gew.-% |
| Schwersieder | 1,1 Gew.-% |

In der ersten Kolonne erfolgte bei einer Sumpftemperatur von 168°C und einer Kopftemperatur von 95°C die Abtrennung der Leichtsieder und des Wassers. Der Feedstrom von der ersten in die zweite Kolonne hatte somit folgende Zusammensetzung:

| | |
|---|---|
| IPDA | 98,7 Gew.-% |
| Schwersieder | 1,3 Gew.-% |

In der zweiten Kolonne erfolgte bei einer Sumpftemperatur von 187°C und einer Kopftemperatur von 159°C die Feinreinigung von IPDA. Es wurde hierbei eine Reinheit von 99,95 Gew.-% erreicht. Der gesamte IPDA-Verlust über das 2-Kolonnensystem betrug 0,2 Gew.-%.
Bei einem gewählten Feedmassenstrom von 2160 kg pro Stunde betrug die erforderliche

| | |
|---|---|
| Heizleistung: | 1184 kW |
| Kühlleistung: | 1232 kW |

### Beispiel 2: Erfindungsgemäße Trennwandkolonne

Die Simulation der Destillation erfolgte über Aspen Plus. Im erfindungsgemäßen Beispiel wurde eine Trennwandkolonne zur Feindestillation von IPDA verwendet. Die Trennwandkolonne wies 36 theoretische Trennstufen auf, wobei im Bereich der Trennstufen 9 - 27 eine Trennwand eingezogen wurde. Es wurde ein Rücklauf-zu-Feed-Verhältnis von 1,1 gewählt, wobei die Flüssigphase zu 66 % und die Dampfphase zu 40 % auf die Produktseite der Trennwandkolonne geführt wurden. Hierbei wurde der Kolonnendruck der Trennwandkolonne auf 80 mbar festgesetzt. Der eingesetzte Feedstrom hatte folgende Zusammensetzung in Gewichts-% (Gew.-%):

| | |
|---|---|
| IPDA | 88,6 Gew.-% |
| Wasser | 9,1 Gew.-% |
| Leichtsieder | 1,2 Gew.-% |
| Schwersieder | 1,1 Gew.-% |

Die Feinreinigung von IPDA erfolgte bei einer Sumpftemperatur von 187°C, einer Kopftemperatur von 95°C und einer Seitenstromtemperatur von 159°C. Es wurde hierbei eine Reinheit von 99,95 Gew.-% erreicht. Der gesamte IPDA-Verlust über die Trennwandkolonne betrug 0,2 Gew.-%.
Bei einem gewählten Feedmassenstrom von 2160 kg pro Stunde betrug die erforderliche

| | |
|---|---|
| Heizleistung: | 678 kW |
| Kühlleistung: | 667 kW |

Die erforderliche Heiz- bzw. Kühlleistung konnte durch den Einsatz einer Trennwandkolonne im Vergleich zum 2-Kolonnensystem um 43 % bzw. 46 % reduziert werden.

## Patentansprüche

1. Verfahren zur Feinreinigung von Isophorondiamin aus der Herstellung von Isophorondiamin durch aminierende Hydrierung von Isophoronnitril in Anwesenheit von mindestens Ammoniak, Wasserstoff, eines Hydrierkatalysators und gegebenenfalls weiteren Zusätzen und in An- oder Abwesenheit von organischen Lösungsmitteln, wobei ein Roh-Isophorondiamin erhalten wird,
**dadurch gekennzeichnet, dass** das Roh-Isophorondiamin in mindestens einer Trennwandkolonne einer Feinreinigung unterworfen wird.

2. Verfahren nach Anspruch 1, **dadurch gekennzeichnet, dass** Roh-Isophorondiamin im Allgemeinen die folgende Zusammensetzung in Gewichts-% (Gew.-%) aufweist:
| | |
|---|---|
| IPDA | 75-100 Gew.-% |
| Wasser | 0-15 Gew.-% |
| Leichtsieder | 0-6 Gew.-% |
| Schwersieder | 0-6 Gew.-%. |

3. Verfahren nach Anspruch 1-2, **dadurch gekennzeichnet, dass** über dem Kopf der Trennwandkolonne die Abtrennung von Wasser und Leichtsiedern erfolgt.

4. Verfahren nach Anspruch 1-2, **dadurch gekennzeichnet, dass** aus dem Sumpf der Trennwandkolonne die Schwersieder abgetrennt werden.

5. Verfahren nach Anspruch 1-2, **dadurch gekennzeichnet, dass** das Rein-Isophorondiamin aus mindestens einem Seitenstrom der Trennwandkolonne gewonnen wird.

6. Verfahren nach Anspruch 1-2, **dadurch gekennzeichnet, dass**
- über dem Kopf der Trennwandkolonne die Abtrennung von Wasser und Leichtsiedern erfolgt,
- aus dem Sumpf der Trennwandkolonne die Schwersieder abgetrennt werden,
- das Rein- Isophorondiamin aus mindestens einem Seitenstrom der Trennwandkolonne gewonnen wird.

7. Verfahren nach Anspruch 1-6, **dadurch gekennzeichnet, dass** die Trennwandkolonne folgende Rahmenbedingungen aufweist:
| | |
|---|---|
| Sumpftemperatur | 120-250°C |
| Kopftemperatur | 40-120 °C |
| Seitenstromtemperatur | 100-200 °C |
| Theoretische Stufen | 10-130 |

8. Verfahren nach Anspruch 1-7, **dadurch gekennzeichnet, dass** das Roh-Isophorondiamin in einer Trennwandkolonne einer Feinreinigung unterworfen wird.
